# EUROPEAN PATENT APPLICATION

(11) **EP 2 261 260 A2**
(43) Date of publication of application: **15.12.2010**
(21) Application number: 10182410.0
(22) Date of filing: 24.03.2005
(51) Int. Cl.: C07K 16/44, A61K 39/40

(54) **Methods for inducing autolysis in infectious bacteria**

(30) Priority: 27.03.2004 GB 0407008
(62) Divisional of application: 05729289.8
(71) Applicant: Haptogen Ltd, Aberdeen AB25 2ZD (GB)
(72) Inventor: Charlton, Keith Alan, Foresterhill Aberdeen AB25 2ZD (GB); Porter, Andrew Justin Radcliffe, Foresterhill Aberdeen AB25 2ZD (GB); Broadbent, Ian, Foresterhill Aberdeen AB25 2ZD (GB)
(74) Representative: Bassil, Nicholas Charles

(57) **Abstract**

The present invention relates to methods for the killing of infectious bacteria by modulating the extra-cellular concentration of bacterial cell signalling molecules. This has the effect of inducing rapid cell death (autolysis) in the majority of bacterial cells, and preventing virulence or restoring a benign state in surviving cells. These receptors have applications for the treatment of individuals with susceptibility to infection, the treatment of patients with existing infections, in disease management, and in related applications where the host for infection is an animal or plant. The compositions described herein are particularly relevant to Pseudomonas aeruginosa infection, for example in the treatment of pulmonary infection in cystic fibrosis patients, and represent a unique bactericidal medication that does not directly target the bacteria.

## Description

### Field of the invention

The present invention relates to methods for controlling and treating bacterial infections in patients. The invention provides for the application of therapies based upon, in the preferred embodiment, immunoglobulin or immunoglobulin-like receptor molecules that have affinity and specificity for acyl homoserine lactone signalling molecules involved in the processes of bacterial cell to cell communication. By binding to such molecules, the receptors can be used to modulate the extra-cellular concentrations of molecules involved in environment-sensing and virulence in *Pseudomonas aeruginosa,* and in so doing can induce a process of rapid cell death (autolysis) within bacterial populations.

### Background of the invention

One of the major causes of mortality and morbidity amongst patients undergoing treatment in hospitals today is due to hospital acquired infection. Susceptibility to such infection can be as a result of the primary illness for which the patient was admitted, of immuno-suppressive treatment regimes, or as a consequence of injury resulting in serious skin damage, such as burns. The bacterium to which the highest proportion of cases is attributed is *Pseudomonas aeruginosa.* It is the epitome of an opportunistic pathogen of humans. The bacterium almost never infects uncompromised tissues, yet there is hardly any tissue that it cannot infect, if the tissue defences are compromised in some manner. Although accounting for a relatively small number of species, it poses a serious threat to human health and is used hereafter as a representative example of an infectious bacterium, and does not in any way limit the scope or extent of the present invention.

*Ps. aeruginosa* is an opportunistic pathogen that causes urinary tract infections, respiratory system infections, dermatitis, soft tissue infections, bacteraemia and a variety of systemic infections, particularly in victims of severe burns, and in cancer and AIDS patients who are immuno-suppressed. Respiratory infections caused by *Ps. aeruginosa* occur almost exclusively in individuals with a compromised lower respiratory tract or a compromised systemic defence mechanism. Primary pneumonia occurs in patients with chronic lung disease and congestive heart failure. Bacteraemic pneumonia commonly occurs in neutropenic cancer patients undergoing chemotherapy. Lower respiratory tract colonisation of cystic fibrosis patients by mucoid strains of *Ps. aeruginosa* is common and difficult, if not impossible, to treat. It causes bacteraemia primarily in immuno-compromised patients. Predisposing conditions include haematologic malignancies, immuno-deficiency relating to AIDS, neutropenia, diabetes mellitus, and severe burns. Most *Pseudomonas* bacteraemia is acquired in hospitals and nursing homes where it accounts for about 25 percent of all hospital acquired gram-negative bacteraemias.

The bacterium is notorious for its natural resistant to many antibiotics due to the permeability barrier afforded by its outer membrane LPS and is, therefore, a particularly dangerous and dreaded pathogen. Also, its tendency to colonise surfaces in a biofilm form makes the cells impervious to therapeutic concentrations of antibiotics. Since its natural habitat is the soil, living in association with the bacilli, actinomycetes and moulds, it has developed resistance to a variety of their naturally occurring antibiotics. Moreover, *Pseudomonas* spp. maintain antibiotic resistance plasmids, both Resistance factors (R-factors) and Resistance Transfer Factors (RTFs), and are able to transfer these genes by means of the bacterial processes of transduction and conjugation. Only a few antibiotics are effective against *Pseudomonas,* including fluoroquinolone, gentamicin and imipenem, and even these antibiotics are not effective against all strains. Combinations of gentamicin and carbenicillin are reportedly effective in patients with acute *Ps. aeruginosa* infections. The futility of treating *Pseudomonas* infections with antibiotics is most dramatically illustrated in cystic fibrosis patients, virtually all of whom eventually become infected with a strain that is so resistant it cannot be treated. Because of antibiotic resistance, susceptibility testing of clinical isolates is mandatory.

*Ps. aeruginosa* can usually be isolated from soil and water, as well as the surfaces of plants and animals. It is found throughout the world, wherever these habitats occur, so it is quite a "cosmopolitan" bacterium. It is sometimes present as part of the normal flora of humans, although the prevalence of colonisation of healthy individuals outside the hospital is relatively low (estimates range from 0 to 24 percent depending on the anatomical locale). In hospitals it is known to colonise food, sinks, taps, mops, respiratory equipment and surgical instruments. Although colonisation usually precedes infections by *Ps. aeruginosa,* the exact source and mode of transmission of the pathogen are often unclear because of its ubiquitous presence in the environment. Amongst intensive care patients in whom infection is suspected on clinical grounds, as many as 50% have no identifiable source for infection. Currently 1,400 deaths worldwide are caused each day by *Ps. aeruginosa* in intensive care units (ICU's), making it the No. 1 killer.

*Ps. aeruginosa* is primarily a nosocomial pathogen. According to the CDC, the overall incidence of *Ps. aeruginosa* infections in US hospitals averages about 0.4 percent (4 per 1000 discharges), and the bacterium is the fourth most commonly isolated nosocomial pathogen accounting for 10.1% of all hospital-acquired infections. Globally it is responsible for 16% of nosocomial pneumonia cases, 12% of acquired urinary tract infections, 8% of surgical wound infections and 10% of bloodstream infections. Immune-compromised patients such as neutropenic cancer and bone marrow transplant patients are susceptible to opportunistic *Ps. aeruginosa* infection, leading to 30% of reported deaths. It is also responsible for 38% of ventilator-associated pneumonias and 50% of deaths amongst AIDS patients. In bums cases *Ps. aeruginosa* infections have declined in recent years due to improved treatment and dietary changes. Mortality rates however remain high, accounting for 60% all deaths due to secondary infection of bums patients.

One reason for the versatility of *Ps. aeruginosa* is that it produces a diverse battery of virulence determinants including elastase, LasA protease, alkaline protease, rhamnolipids, type IV pilus-mediated twitching motility, pyoverdin (Williams et al., 1996, Stintzi et al., 1998, Glessner et al., 1999), pyocyanin (Brint & Ohman, 1995, Reimmann et al., 1997) and the cytotoxic lectins PA-I and PA-II (Winzer et al., 2000). It is now known that many of these virulence determinants are regulated at the genetic level in a cell density-dependent manner through quorum sensing. *Ps. aeruginosa* possesses two well characterised quorum sensing systems, namely the *las* and *rhl* (*vsm*) systems which comprise of the LuxRI homologues LasRI (Gambello & Iglewski, 1991) and RhlRI (VsmRI) (Latifi et al., 1995) respectively. LasI directs the synthesis of 3-oxo-C12-HSL (Passador et al., 1993, Pearson et al., 1994) whereas RhlI directs the synthesis of C4-HSL (Winson et al., 1995). The *las* and the *rhl* systems are thought to exist in a hierarchy where the *las* system exerts transcriptional control over RhlR (Williams et al., 1996, Pesci et al., 1997). The transcriptional activator LasR functions in conjunction with 3-oxo-C12-HSL to regulate the expression of the genes encoding for the virulence determinants elastase, LasA protease, alkaline protease and exotoxin A (Gambello & Iglewski, 1991, Toder et al., 1991, Gambello et al., 1993, Pearson et al., 1994) as well as *lasI.* Elastase is able to cleave collagen, IgG and IgA antibodies, complement, and facilitates bacterial adhesion onto lung mucosa. In combination with alkaline protease it also causes inactivation of gamma Interferon (INF) and Tumour Necrosis Factor (TNF). LasI directs the synthesis of 3-oxo-C12-HSL which together with LasR, binds to the *lasI* promoter and creates a positive feedback system. The RhlR transcriptional activator, along with its cognate AHL (C4-HSL), regulates the expression of *rhlAB* (rhamnolipid), *lasB*, *aprA*, RpoS, cyanide, pyocyanin and the lectins PA-I and PA-II (Ochsner et al., 1994, Brint & Ohman, 1995, Latifi et al., 1995, Pearson et al., 1995, Winson et al., 1995, Latifi et al., 1996, Winzer et al., 2000). These exist in a hierarchical manner where by the LasR/3-oxo-C12-HSL regulates *rhlR* (Latifi et al., 1996, Pesci et al., 1997) and consequently both systems are required for the regulation of all the above virulence determinants.

More recently another class of signal molecule involved in the quorum sensing systems of *Ps. aeruginosa* have been identified. These are a group of related molecules based on 4-hydoxy-2-alkylquinolines (HAQ's), many of which show anti-bacterial activity. First identified in 1959, and later characterised (Pesci *et al,* 1999), 2-heptyl-3-hydroxy-4-quinoline was designated PQS (*Pseudomonas* quinolone signal) for its role in quorum sensing. Later research has revealed the structures of a series of HAQ's that are synthesised and secreted by *Ps. aeruginosa*, some of which are also implicated in cell-to-cell communication systems. One of these, 2-heptyl-4-hydoxyquinoline (HHQ) is thought to be secreted by cells during growth phase, taken up by adjacent cells and converted into PQS at late growth/early stationary phase, and subsequently released as a signal molecule throughout stationary phase (Déziel *et al*., 2004). This coincides with the production of several quorum-sensing regulated virulence factors such as pyocyanin and elastase (Diggle *et al*., 2003). Confirmation of the involvement of PQS was obtained from the observation that addition of exogenous PQS to the growth medium during early growth phase resulted in early production of these virulence factors. The fact that peak exogenous PQS levels are found in late stationary phase suggests that it is not involved in cell-density sensing.

The activity of PQS is very closely linked to the previously described quorum sensing system of *Ps. aeruginasa.* Its synthesis is regulated by both *las* and *rhl*, the former being responsible for the induction of PQS, and the latter system able to repress PQS (McGrath *et al.* 2004). Moreover, PQS production has also been found to be dependant on the ratio of the AHL signal molecules produced by the other systems, i.e. 3-oxo-C12-HSL and C4-HSL. PQS is able to stimulate production of the virulence factor elastase and also induces the expression of *rhlI*, which in turn encodes the C4-HSL synthase (McKnight *et al*., 2000). Furthermore, the bioactivity of PQS is dependant on the presence of RhlR (Pesci *et al*., 1999), and it therefore seen as an important component of the hierarchical regulation of quorum sensing.

One of the most serious clinical conditions induced by *Ps. aeruginosa* is the destructive chronic lung infection of cystic fibrosis (CF) sufferers. Almost all patients' lungs are infected by the age of three years (Bums *et al*., 2001). The immune systems of CF patients are unable to clear the bacteria, resulting in the onset of chronic disease with the associated extensive tissue damage and airway blockage from which the majority of patients eventually succumb. The establishment and persistence of *Ps. aeruginosa* lung infection has long been associated with the development of a biofilm phenotype, in addition to induction of other quorum-sensing regulated virulence factors (Singh *et al*., 2000). Quorum sensing signals are readily detected in CF lung of infected mice (Wu *et al*., 2000). Amongst other effects, the production of the well characterised AHL signalling molecules by *Ps. aeruginosa* in the lung can directly affect host immune responses by modulating the isotype ratio of the antibody response and cytokine levels (Wu *et al*., 2004).

A recent study of mutants created from the *Ps. aeruginosa* clinical isolate PA01 identified strains that underwent autolysis (programmed cell death) under conditions of high cell density (D'Argenio *et al*., 2002). Detailed analysis of a number of these strains revealed that all contained mutations that resulted in the over production of the PQS quorum sensing signal. The subsequent introduction of a second mutation that reduced levels of secreted PQS, restored the wild type phenotype (i.e. prevented or greatly reduced autolysis), thus confirming the involvement of PQS in the observed cell death. (Guina *et al*., 2003) report that the production of PQS by *Ps. aeruginosa* can also be induced by growth in magnesium-limiting medium. Together with expression of other stress-response genes, PQS synthesis in low magnesium indicates a response to starvation. These conditions would be similar to those found in host lung environments, and induction of virulence factors would assist the bacteria in fighting the host immune system. The autolysis effect seen by D'Argenio *et al*., where the majority but not all cells died, resulted from excessive rather than regulated production of PQS, and could mimic an extreme response to adverse conditions. In such situations, it would benefit the bacteria to reduce its numbers significantly in order to permit survival of the few (D'Argenio *et al*., 2002).

A number of different approaches are being actively pursued to develop therapeutics for the treatment or prevention of *Ps. aeruginosa* infection. Some are intended to be broad ranging while others are directed at specific types of *Pseudomonas* infection. Those that follow traditional routes include the development of vaccines such as that described in US patent 6,309,651, and a new antibiotic drug (SLIT) that is hoped will be effective against gram-negative bacteria in general but is designed primarily to act against *Ps. aeruginosa* and is administered by aerosol inhalation. A further observation under investigation is that the antibiotic erythromycin administered at sub-optimal growth inhibitory concentrations simultaneously suppresses the production of *Ps. aeruginosa* haemagglutinins, haemolysin, proteases and acyl-homoserine lactones, and may be applicable for the treatment of persistent *Ps. aeruginosa* infection. Cream formulations containing amphipathic peptides are also being examined as a possible means of preventing infection of bums or other serious skin wounds. US patent 6,309,651 also teaches that antibodies against the PcrV virulence protein of *Ps. aeruginosa* may afford protection against infection.

There is also some interest in the modulation of homoserine lactone levels as a means of controlling pathogenicity. Certain algae have been demonstrated to produce competitive inhibitors of acyl-homoserine lactones such as furanones (Manefield, 1999), as have some terrestrial plants. These compounds displace the AHL signal molecule from its receptor protein and can act as agonist or antagonist in AHL bioassays (Tepletski et al., 2000). Other methods employed to reduce AHL concentration include the development of auto-inducer inactivation enzymes (AiiA's) that catalyse the degradation of AHLs and the sequestering of AHL by antibodies (WO 2004/014423).

There are a number of potential problems and limitations associated with the therapies currently under development. It is as yet unproven as to whether vaccines will be efficacious treatments. *Ps. aeruginosa* produces an extensive mucoid capsule that effectively protects against opsonisation by host antibodies, as revealed by patients with persistent infections having high serum titres of anti-*Pseudomonas* antibodies. The use of auto-inducer mimics are limited by the concentrations of most that are required to effectively compete against AHLs for the receptor binding site, and the possibility of side effects. It is well known that AHLs released by *Pseudomonas* and other bacteria have a number of direct effects on human physiology. These include inhibition of histamine release as described in WO 01/26650. WO 01/74801 describes that AHLs are also able to inhibit lymphocyte proliferation and down-regulate the secretion of TNF-α by monocytes and macrophages, so acting as a general immuno-suppressant. There is a danger therefore that therapies involving the use of competitive AHL mimics may result in down-regulation of the patient's immune system. This would be generally undesirable, and particularly so in immuno-compromised patients. The use of antibiotics can, at best, be viewed as a short-term strategy in view of the remarkable ability of this bacterium (and others) to develop resistance to antibiotics.

That the pathogenesis of *Ps. aeruginosa* is clearly multifactoral is underlined by the large number of virulence factors and the broad spectrum of diseases associated with this bacterium. Many of the extra-cellular virulence factors required for tissue invasion and dissemination are controlled by cell-to-cell signalling systems involving homoserine lactone-based and PQS signal molecules and specific transcriptional activator proteins. These regulatory systems allow *Ps. aeruginosa* to adapt to a virulent form in a co-ordinated cell density dependent manner, and to overcome host defence mechanisms. They are also used to regulate the population of bacteria, and specifically to reduce bacterial numbers, under conditions where essential nutrients are limiting such that the remaining bacteria have a survival advantage. Interference with such cell signalling systems in order to induce autolysis is a promising therapeutic approach to reducing illness and death caused by *Ps. aeruginosa.*

There is a need to develop effective means of modulating the concentrations of HSLs and other bacterial cell signalling molecules involved in pathogenicity by methods that do not have adverse side effects, and are unlikely to be evaded by pathogenic bacteria in the foreseeable future. A composition or compound capable of killing bacteria, particularly *Pseudomonas aeruginosa,* that did not attack the bacterial cell directly and so is unlikely to lead to resistant strains would be of considerable benefit to the treatment of disease states such as CF. The present invention provides for such compositions.

### Summary of the invention

The present invention provides for methods for reducing numbers of the pathogenic bacterium *Pseudomonas aeruginosa* by regulating the extra-cellular concentrations of bacterial cell signalling molecules. By selective removal (binding or degradation) of lactone-derived cell signal molecules, an imbalance in the ratios of AHL to PQS signal molecules is produced which stimulates rapid cell death (or autolysis) of *Ps. aeruginosa.* Alternatively, PQS may be administered alone or in conjunction with anti-AHL receptors. Whereas other bactericidal treatments act directly on the cell to cause death, the present invention targets extra-cellular signalling molecules in order to mimic an environment unable to sustain high population densities, and so induce a collapse in bacterial cell numbers. As such it is much less likely that strains resistant to the therapy will emerge.

According to a first aspect of the present invention, there is provided a method of causing autolysis of a population of gram-negative bacteria, said method comprising administration to the population of an antibody to a lactone or lactone-derived signal molecule secreted by gram-negative bacteria so as to cause an imbalance in the ratio of homoserine lactone (HL) signal molecule to quinolone signal (QS) signal molecule in the environment of the population of the gram-negative bacteria.

The gram-negative bacteria may be *Actinobacillus actinomycetemcomitans*, *Acinetobacter baumannii, Bordetella pertussis, Brucella* sp., *Campylobacter* sp., *Capnocytophaga* sp., *Cardiobacterium hominis, Eikenella corrodens, Francisella tularensis, Haemophilus ducreyi, Haemophilus influenzae, Helicobacter pylori, Kingella kingae, Legionella pneumophila, Pasteurella multocida, Citrobacter* sp., *Enterobacter* sp., *Escherichia coli, Klebsiella pneumoniae, Proteus* sp., *Salmonella enteriditis, Salmonella typhi, Serratia marcescens, Shigella* sp., *Yersinia enterocolitica, Yersinia pestis, Neisseria gonorrhoeae, Neisseria meningitidis, Moraxella catarrhalas, Veillonella* sp., *Bacteroides fragilis, Bacteroides* sp., *Prevotella* sp., *Fusobacterium* sp., *Spirillum minus, Aeromonas* sp., *Plesiomonas shigelloides, Vibrio cholera, Vibrio parahaemolyticus, Vibrio vulnificus, Acinetobacter* sp., *Flavobacterium* sp., *Pseudomonas aeruginosa, Burkholderia cepacia, Burkholderia pseudomallei, Xanthomonas maltophilia, or Stenotrophomonas maltophila*. In a preferred embodiment, the gram-negative bacteria is *Pseudomonas aeruginosa.*

Suitably, the homoserine lactone (HL) signal molecule may be a homoserine lactone molecule with a formula selected from the group consisting of: 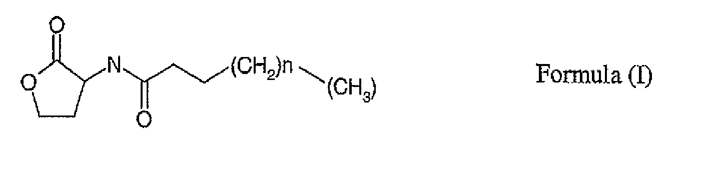 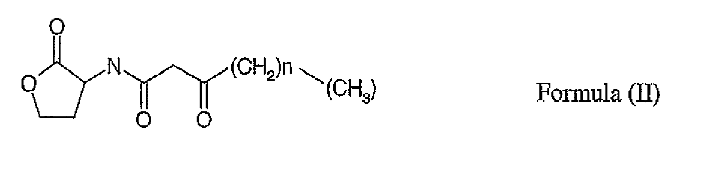 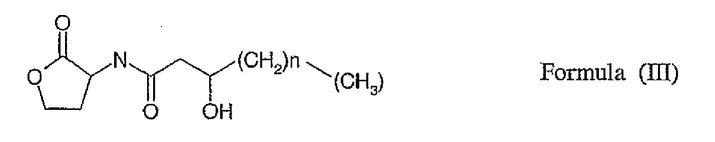 where n may be from 0 to 12.

The homoserine lactone molecule of general formula (I) may be N-butanoyl-L-homoserine lactone (BHL) where n = 0, *N*-dodecanoyl-L-homoserine lactone (dDHL) where n = 8 or *n*-tetradecanoyl-L-homoserine lactone (tDHL) where n = 10.

The homoserine lactone molecule of general formula (II) may be *N*-(-3-oxododecanoyl)-L-homoserine lactone (OdDHL) where n = 8 or N-(-3-oxohexanoyl)-L-homoserine lactone (OHHL) where n = 2.

The homoserine lactone molecule of general formula (III) may be *N*-(-3-hydroxybutanoyl)-L-homoserine lactone (HBHL) where n = 0.

The lactone signal molecule may be any acyl-homoserine signal molecule, and is preferably OdDHL and/or BHL.

The quinolone signal (QS) signal molecule may be a molecule of general formula (IV) 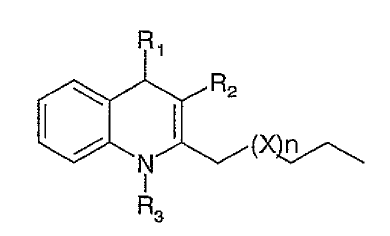
where n may be 1 to 7,
R₁ may be =O, or -H,
R₂ may be -OH, or -H, and
R₃ may be -H, or alternatively, the nitrogen atom (N) may be unsubsituted, in which case the aromatic ring is further unsaturated.

Suitably, the quinolone signal molecule of general formula (IV) may be

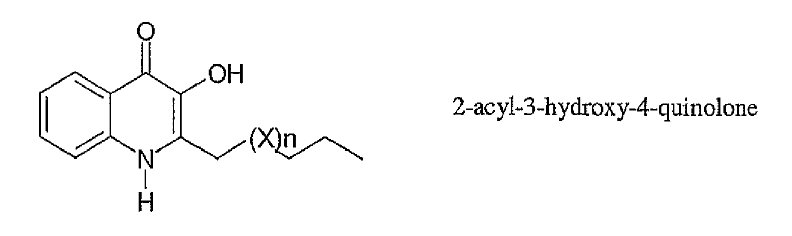

Preferably, the QS molecule is *Pseudomonas* quinolone signal (PQS) or 2-heptyl-3-hydroxy-4-quinolone 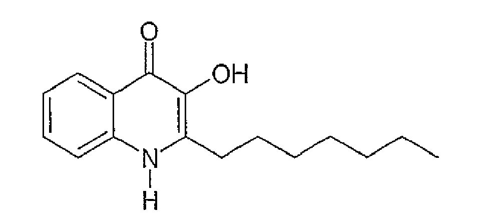 2-heptyl-3-hydroxy-4-quinolone

In one embodiment of the invention, the gram negative bacteria may *Pseudomonas aeruginosa* and the ratio of bacterial signal molecules may be acyl-homoserine lactone (AHL) signal molecule of formula (I) to *Pseudomonas* quinolone signal (PQS) molecule.

A growing number of bacterial species are being found to communicate between cells using a variety of small signal molecules. Gram-negative bacteria predominantly use *N*-acyl homoserine lactones. The latter are a group of compounds that share a common homoserine lactone ring structure and vary in the length and structure of a side chain. There are three classes within the group, the acyl-homoserine lactones, the 3-oxo-homoserine lactones and the 3-hydroxy-homoserine lactones. A single species can produce and respond to members of more than one class. *Pseudomonas aeruginosa* uses *N*-butyryl-homoserine lactone (BHL), 3-oxo-dodecanoyl-homoserine lactone (OdDHL) and the *Pseudomonas* quinolone signal 2-heptyl-3-hydroxy-4-quinolone (PQS).

The cells use the molecules as a means of determining the local cell density, such that in conditions of low cell density the concentration of signal molecule is correspondingly low. In high cell densities the local signal molecule concentration is high. When this concentration reaches a threshold level it induces the transcription of genes involved in virulence and the onset of a disease state in the host.

Bacterial signalling molecules are being discovered in every organism for which they are searched. It seems to be a ubiquitous system, applicable to every species. The main differences are that all gram negative (gram -ve) bacteria use homoserine lactone-based molecules, and gram positive (gram +ve) bacteria use (modified) small peptides. *Ps. aeruginosa* is an example of a gram negative bacteria which uses two signal molecules AHL and PQS.

Previous work in this field has concentrated on mimicking signal molecules with ones that are recognised but that do not function, i.e. no pathogenic switching, or on blocking the various receptor systems. The disadvantages of these methods are principally that resistance can be developed to the mimic or block and the 'real' signal molecule is still there and will compete for binding. In addition, some bacterial signalling molecules e.g. acyl-homoserine lactones are virulence factors in their own right, and can directly cause imanuno-suppression of the host (i.e. patient).

The present invention provides for methods which use antibodies that target the actual signal molecule rather than the cell itself. This approach has a key and important advantage over all previous efforts in the field in that the bacteria will not recognise that they are being attacked, they will simply detect that that they are alone. There will not be any selective pressure for resistance. The aspects of present invention are further advantageous in that they are able to bring about bacterial killing by inducing an endogenous system of programmed cell death. A bactericidal treatment that does not directly target bacterial cells represents a significant departure from existing medications.

Antibodies according to the present invention can be polyclonal antibodies or monoclonal antibodies. Polyclonal antibodies can be raised by stimulating their production in a suitable animal host (e.g. a mouse, rat, guinea pig, rabbit, sheep, chicken, goat or monkey) when the antigen is injected into the animal. If necessary an adjuvant may be administered together with the antigen. The antibodies can then be purified by virtue of their binding to antigen or as described further below. Monoclonal antibodies can be produced from hybridomas. These can be formed by fusing myeloma cells and B-lymphocyte cells which produce the desired antibody in order to form an immortal cell line. This is the well known Kohler & Milstein technique (Nature 256 52-55 (1975)).

Techniques for producing monoclonal and polyclonal antibodies which bind to a particular protein are now well developed in the art. They are discussed in standard immunology textbooks, for example in Roitt et at, Immunology second edition (1989), Churchill Livingstone, London.

In addition to whole antibodies, the present invention includes derivatives thereof which are capable of binding to antigen. Thus the present invention includes antibody fragments and synthetic constructs. Examples of antibody fragments and synthetic constructs are given by Dougall et al in Tibtech 12 372-379 (September 1994). Antibody fragments include, for example, Fab, F(ab')₂ and Fv fragments (see Roitt et al [supra]). Fv fragments can be modified to produce a synthetic construct known as a single chain Fv (scFv) molecule. This includes a peptide linker covalently joining V_{H} and V_{L} regions which contribute to the stability of the molecule. The present invention therefore also extends to single chain antibodies or scAbs.

Other synthetic constructs include CDR peptides. These are synthetic peptides comprising antigen binding determinants. Peptide mimetics may also be used. These molecules are usually conformationally restricted organic rings which mimic the structure of a CDR loop and which include antigen-interactive side chains. Synthetic constructs also include chimaeric molecules. Thus, for example, humanised (or primatised) antibodies or derivatives thereof are within the scope of the present invention. An example of a humanised antibody is an antibody having human framework regions, but rodent hypervariable regions. Synthetic constructs also include molecules comprising a covalently linked moiety which provides the molecule with some desirable property in addition to antigen binding. For example the moiety may be a label (e.g. a detectable label, such as a fluorescent or radioactive label) or a pharmaceutically active agent.

In order to generate anti-bacterial signal molecule antibodies, it is preferable to conjugate the target molecule, or a suitable derivative, to two different carrier molecules (proteins), though a single conjugated species can be also used. Bacterial signal molecules, in general, are too small to stimulate an immune response *in-vivo*, or to be used directly as a source of antigen for the selection of high affinity antibodies from antibody libraries. Selection of antibodies specific for the cell signalling molecular (hereafter referred to as 'antigen') is carried out in the preferred embodiment using a repertoire (library) of first members of specific binding pairs (sbp), for example a library of antibodies displayed on the surface of filamentous bacteriophage. Any other system that allows for the selection of specific receptors from a library of receptors is also applicable for the methods of the present invention. In alternative embodiments signal molecule-specific clones can be selected from a panel of antibody secreting hybridoma cell lines generated from an animal immunised with an antigen conjugate. For the purposes of a general illustration the example of a library of antibody binding sites displayed on phage particles will be used.

A conjugate comprising an antigen coupled to a suitable carrier molecule, which can be a protein, a peptide or any natural or synthetic compound or material (referred to hereafter as 'conjugate-1') is immobilised onto a suitable solid support such as an 'immunotube' or microtitre plate, and the uncoated surface blocked with a nonspecific blocking agent such as dried milk powder. Suitable conjugate molecules can include, but are not limited to proteins such as bovine serum albumin (BSA), Keyhole Limpet Haemocyanin (KLH), Bovine Thyroglobulin (TG), Ovalbumin (Ova), or non-proteins such as biotin. The only restriction on the selection of the conjugate molecule is that it be immobilisable in some way and for immunisation is large enough to elicit an immune response.

A library of first members of specific binding pairs (sbp's) ('the library') is applied to the immobilised conjugate and incubated for sufficient time for sbp members recognising conjugate-1 to bind. Phage not recognising the conjugate are removed by stringent washing. Phage that remain bound are eluted, for example with triethylamine or other suitable reagent, into a buffer solution to restore neutral pH. Recovered phage particles are then infected into a suitable host organism, e.g. *E. coli* bacteria, and cultured to amplify numbers of each selected member and so generate a second 'enriched' library. The process is then repeated using the enriched library to select for phage-antibodies ('phage') recognising the antigen conjugated to a second carrier protein (conjugate-2).

Additional rounds are performed as required, the selection process being altered to favour selection of those sbp members recognising the free form of the antigen. Phage are selected against antigen conjugates as described previously, using initially conjugate-1, and alternating with conjugate-2 (where available) for each subsequent round. Bound phage are eluted by incubating with a solution of free antigen, or antigen conjugated to small soluble selectable moieties, e.g. biotin, for sufficient time for sbp members with higher affinity for the bound form of the antigen to dissociate from the immobilised conjugate. Those phage eluted with free antigen are infected into *E. coli* cells for amplification and re-selection, and those remaining bound to the immobilised antigen discarded. Alternatively, but less preferably, all antibodies binding to conjugate may be eluted e.g. with low pH.

Individual (monoclonal) phage clones from each round of selection are screened for desired binding characteristics. This can be performed by a variety of methods that will be familiar to those with ordinary skill in the art, depending on requirements, including such techniques as SPR (Surface Plasmon Resonance) and ELISA (Enzyme Linked Immuno-Sorbant Assay). Selection criteria will include the ability to bind preferentially to the free soluble form of the antigen in the presence of conjugated derivatives.

In the preferred embodiment of the invention, antibodies will be generated from a naïve human antibody phage display library (McCafferty et al., Nature 348: 552-554, 1990; and as described in WO 92/01047). Thus the antibodies can be used for administering to patients without eliciting an immune response. In other embodiments a library can be constructed from an animal pre-immunised with one or more conjugates of an AHL and a suitable carrier molecule. A further alternative is the generation of hybridoma cell lines from an animal immunised as described above. In the latter two cases it is preferable that steps be taken to reduce the immunogenicity of resulting antibodies, for example by creating host animal-human chimaeric antibodies, or "humanisation" by CDR grafting onto a suitable antibody framework scaffold. Other methods applicable will include the identification of potential T-cell epitopes within the antibody, and the subsequent removal of these e.g. by site-directed mutagenesis (de-immunisation). In a further embodiment the antibody can be engineered to include constant regions from different classes of human immunoglobulin (IgG, IgA, etc.) and produced as a whole antibody molecule in animal cells. In particular these approaches are desirable where the antibodies are to be used therapeutically. The use of secretory IgA isotype antibodies may be preferable where intra-nasal/aerosol application is envisaged for example in the treatment of *Ps. aeruginosa* infections of cystic fibrosis patients.

For the present invention, the antibody may be monoclonal or polyclonal. The antibodies may be human or humanised. Antibody fragments or derivatives, such as Fab, F(ab').sup.2 (also written as F(ab')₂), Fv, or scFv, may be used, as may single-chain antibodies (scAb) such as described by Huston et al. (Int. Rev. Immunol. 10: 195-217, 1993), domain antibodies (dAbs), for example a single domain antibody, or antibody-like single domain antigen-binding receptors. In addition to antibodies, antibody fragments and immunoglobulin-like molecules, peptidomimetics or non-peptide mimetics can be designed to mimic the binding activity of antibodies and induce bacterial cell lysis (rapid cell death) by modulating the extra-cellular ratio of bacterial signal molecules, suitably the ratio of AHL and PQS signal molecules.

In certain preferred embodiments of the invention, the antibodies are scAbs, in particular scAbs that are obtained from *E*. *coli* clones designated as XL1-Blue G3H5, G3B12, G3G2 and/or G3H3. The clones have been deposited at NCIMB, Aberdeen, UK on 18 March 2003 under the terms of The Budapest Treaty under the following accession numbers: G3H5 deposited as NCIMB-41167, G3B12 deposited as NCTMB-41168, G3G2 deposited as NCIMB-41169 and G3H3 deposited as NCIMB-41170. The strains may be cultivated in an appropriate growth media such as LB media supplemented with 100µg/ml ampicillin, optionally supplemented with 12.5µg/ml tetracycline, and/or 1% glucose, under standard conditions of 37°C in air.

### Antibody G3B12 is referred to herein as Hap 2 and antibody G3G2 is referred to herein as Hap 5

After the preparation of a suitable antibody, it may be isolated or purified by one of several techniques commonly available (for example, as described in Antibodies: A Laboratory Manual, Harlow and Lane, eds. Cold Spring Harbor Laboratory Press (1988)). Generally suitable techniques include peptide or protein affinity columns, HPLC or RP-HPLC, purification on Protein A or Protein G columns, or combinations of these techniques. Recombinant antibodies can be prepared according to standard methods, and assayed for specificity using procedures generally available, including ELISA, dot-blot assays etc.

The methods of the present invention provide a means by which a massive crash in the bacterial population can be induced without actually targeting the bacteria directly, i.e. by targeting extra-cellular signalling molecules. Such an approach is without precedent. The invention provides for an approach to bacterial infection which does not lead to the development of resistance from the bacteria.

According to a second aspect of the invention, there is provided a method for the treatment of an infection of gram-negative bacteria in a subject, said method comprising administration to the subject of an antibody to a lactone or lactone-derived signal molecule secreted by gram-negative bacteria so as to cause an imbalance in the ratio of homoserine lactone (HL) signal molecule to quinolone signal (QS) signal molecule in the environment of the gram-negative bacteria. The treatment may be prophylactic or may be in respect of an existing condition.

In such methods, the antibody may be formulated as a pharmaceutical composition according to techniques known in the field of medicine for example by admixing the active ingredient with a carrier(s), diluent (s) or excipient(s) under sterile conditions.

The pharmaceutical composition may be adapted for administration by any appropriate route, for example by the oral (including buccal or sublingual), rectal, nasal, topical (including buccal, sublingual or transdermal), vaginal or parenteral (including subcutaneous, intramuscular, intravenous or intradermal) route. Such compositions may be prepared by any method known in the art of pharmacy, for example by admixing the active ingredient with the carrier(s) or excipient(s) under sterile conditions. Pharmaceutical compositions adapted for oral administration may be presented as discrete units such as capsules or tablets; as powders or granules; as solutions, syrups or suspensions (in aqueous or non-aqueous liquids; or as edible foams or whips; or as emulsions)

Suitable excipients for tablets or hard gelatine capsules include lactose, maize starch or derivatives thereof, stearic acid or salts thereof. Suitable excipients for use with soft gelatine capsules include for example vegetable oils, waxes, fats, semi-solid, or liquid polyols etc.

For the preparation of solutions and syrups, excipients which may be used include for example water, polyols and sugars. For the preparation of suspensions oils (e.g. vegetable oils) may be used to provide oil-in-water or water in oil suspensions.

Pharmaceutical compositions adapted for transdermal administration may be presented as discrete patches intended to remain in intimate contact with the epidermis of the recipient for a prolonged period of time. For example, the active ingredient may be delivered from the patch by iontophoresis as generally described in Pharmaceutical Research, 3 (6), page 318 (1986).

Pharmaceutical compositions adapted for topical administration may be formulated as ointments, creams, suspensions, lotions, powders, solutions, pastes, gels, sprays, aerosols or oils. For infections of the eye or other external tissues, for example mouth and skin, the compositions are preferably applied as a topical ointment or cream. When formulated in an ointment, the active ingredient may be employed with either a paraffinic or a water-miscible ointment base. Alternatively, the active ingredient may be formulated in a cream with an oil-in-water cream base or a water-in-oil base. Pharmaceutical compositions adapted for topical administration to the eye include eye drops wherein the active ingredient is dissolved or suspended in a suitable carrier, especially an aqueous solvent. Pharmaceutical compositions adapted for topical administration in the mouth include lozenges, pastilles and mouth washes. Pharmaceutical compositions adapted for rectal administration may be presented as suppositories or enemas.

Pharmaceutical compositions adapted for nasal administration wherein the carrier is a solid include a coarse powder having a particle size for example in the range 20 to 500 microns which is administered in the manner in which snuff is taken, i.e. by rapid inhalation through the nasal passage from a container of the powder held close up to the nose. Suitable compositions wherein the carrier is a liquid, for administration as a nasal spray or as nasal drops, include aqueous or oil solutions of the active ingredient.

Pharmaceutical compositions adapted for administration by inhalation include fine particle dusts or mists which may be generated by means of various types of metered dose pressurised aerosols, nebulizers or insufflators.

Pharmaceutical compositions adapted for vaginal administration may be presented as pessaries, tampons, creams, gels, pastes, foams or spray formulations.

Pharmaceutical compositions adapted for parenteral administration include aqueous and non-aqueous sterile injection solution which may contain anti-oxidants, buffers, bacteriostats and solutes which render the formulation substantially isotonic with the blood of the intended recipient; and aqueous and non-aqueous sterile suspensions which may include suspending agents and thickening agents. Excipients which may be used for injectable solutions include water, alcohols, polyols, glycerine and vegetable oils, for example. The compositions may be presented in unit-dose or multi-dose containers, for example sealed ampoules and vials, and may be stored in a freeze-dried (lyophilized) condition requiring only the addition of the sterile liquid carried, for example water for injections, immediately prior to use. Extemporaneous injection solutions and suspensions may be prepared from sterile powders, granules and tablets.

The pharmaceutical compositions may contain preserving agents, solubilising agents, stabilising agents, wetting agents, emulsifiers, sweeteners, colourants, odourants, salts (substances of the present invention may themselves be provided in the form of a pharmaceutically acceptable salt), buffers, coating agents or antioxidants. They may also contain therapeutically active agents in addition to the substance of the present invention.

Dosages of the pharmaceutical compositions of the present invention can vary between wide limits, depending upon the disease or disorder to be treated, the age and condition of the individual to be treated, etc. and a physician will ultimately determine appropriate dosages to be used.

Such compositions may be formulated for human or for veterinary medicine. The present application should be interpreted as applying equally to humans as well as to animals, unless the context clearly implies otherwise.

According to a third aspect of the present invention, there is provided an antibody to a lactone or lactone-derived signal molecule secreted by gram-negative bacteria for use in causing autolysis of gram-negative bacteria.

According to a fourth aspect of the invention, there is provided the use of an antibody to a lactone or lactone-derived signal molecule secreted by gram-negative bacteria in the preparation of a medicament for the treatment of an infection of gram-negative in a subject, in which the antibody causes autolysis of the gram-negative bacteria which infect said subject. The treatment may be prophylactic or may be in respect of an existing condition.

The antibody will usually be supplied as part of a sterile, pharmaceutical composition which will normally include a pharmaceutically acceptable carrier, as described above. This pharmaceutical composition may be in any suitable form, (depending upon the desired method of administering it to a patient), as described above.

It may be provided in unit dosage form, will generally be provided in a sealed container and may be provided as part of a kit. Such a kit of parts would normally (although not necessarily) include instructions for use. It may include a plurality of said unit dosage forms.

The methods of the invention can be applied to short or long-term, acute or chronic illness/disease caused by the pathogen *Pseudomonas aeruginosa,* and is of particular concern with patients suffering from cystic fibrosis. Furthermore, as the methods of the invention are directed particularly at bacterial cell signalling molecules, and not primarily at the bacterial cells themselves, there will be no selective pressure exerted on bacterial populations to develop resistance to the treatments described.

The antibody may be administered to infected patients in order to modulate and reduce bacterial infection by bacterial killing, and to reduce the pathogenicity of survivors. This can include inhalation of the antibody in an aerosol by cystic fibrosis patients to increase life expectancy.

In yet another embodiment conjugates of cell signalling molecules to immunogenic proteins can be administered to individuals or patients in order to stimulate an immune response against the lactone signalling molecule resulting in the generation of neutralising antibodies.

In yet another embodiment alternative methods can be applied to the removal of bacterial cell-cell signalling molecules from the blood of a patient with a view to modulating the pathogenicity and virulence of infecting micro-organisms, and reducing bacterial loads by inducing programmed cell death. This can be achieved with other natural receptors or molecules based on natural molecules that bind to said lactone signal molecules. Alternatively non-natural receptors can be applied such as molecularly imprinted polymers (MIPs). This class of receptor have already been shown to be able to bind specifically to small molecular weight bio-molecules such as drugs (Hart et al., 2000) and steroids (Whitcombe et al.,1995; Ramstrom et al., 1996; Rachkov et al., 2000).

In yet another embodiment the receptor may have catalytic or enzymatic activity, and be able to convert the lactone cell signalling molecule into a form that is no longer recognised by the target organism, which then perceives an excess of PQS signal molecule relative to AHL (lactone) signal molecules.

In yet another embodiment the antibody is used in one or more of the above applications in combination, or in combination with other therapies, for example antibiotics, to provide additive and enhanced therapeutic regimes and treatment management.

The antibodies (or equivalent) of the present invention could be administered to treat bacterial infection, or used as a preventative measure for those at high risk of infection. In the case where infection already exists, the antibodies may be administered alone or in combination with anti-bacterial antibodies or antibiotics or other anti-microbial treatments. Administration of anti-AHL antibodies in conjunction with other therapies may allow the use of shorter courses or lower doses of therapeutics, so decreasing the risk of resistance arising and improving patient compliance.

Preferred features for the second and subsequent aspects of the invention are as for the first aspect *mutatis mutandis.*

In one preferred embodiment, there is provided a method of causing autolysis of a population of *Pseudomonas aeruginosa* bacteria, said method comprising administration to the population of an antibody to a lactone or lactone-derived signal molecule secreted by *Pseudomonas aeruginosa* bacteria so as to cause an imbalance in the ratio of acyl-homoserine lactone (AHL) signal molecule to *Pseudomonas* quinolone signal (PQS) signal molecule in the environment of the population of the *Pseudomonas aeruginosa* bacteria.

In one preferred embodiment of the invention, there is provided a method for the treatment of an infection of *Pseudomonas aeruginosa* bacteria in a subject, said method comprising administration to the subject of an antibody to a lactone or lactone-derived signal molecule secreted by *Pseudomonas aeruginosa* bacteria so as to cause an imbalance in the ratio of acyl-homoserine lactone (AHL) signal molecule to *Pseudomonas* quinolone signal (PQS) signal molecule in the environment of the *Pseudomonas aeruginosa* bacteria.

In such embodiments, the acyl-homoserine lactone (AHL) signal molecule may be OdDHL and/or BHL. The antibodies may be scAbs G3H5, G3B 12, G3G2 and/or G3H3 (G3B12 is referred to herein as Hap 2 and antibody G3G2 is referred to herein as Hap 5).

Other objects, features and advantages of the present invention, including but not limited to related applications in plant and animal hosts, will be apparent to those skilled in the art after review of the specification and claims of the invention.

It will be apparent to those of ordinary skill in the art that the compositions and methods disclosed herein may have application across a wide range of organisms in inhibiting, modulating, treating or diagnosing disease or conditions resulting from infection. The compositions and methods of the present invention are described with reference to *Pseudomonas aeruginosa*, but it is within the competence of one of ordinary skill in the art to apply the objects herein to other species.

The invention will now be further described by reference to the non-limiting example and figures detailed below.

### Description of Figures

Figure 1 shows a competition ELISA of the anti-AHL single-chain antibodies Hap2 and Hap 5 binding to dDHL-BSA in the presence of free dDHL and BHL respectively.
Figure 2 shows the dramatic reduction of viable animal-passaged *Ps. aeruginosa* PA01 in the presence of single-chain antibody Hap2 on ice over 40 minutes. The reduction is in the order of 1.5 log CFU/ml.
Figure 3 shows that the survival of non animal-passaged *Ps. aeruginosa* strain PA14 in the presence of monoclonal single-chain antibodies Hap2 or Hap5 compared to a PBS control.
Figure 4 shows bacterial loads of viable *P. aeruginosa* within the bloodstream from tail bleeds taken at 24 h post infection during survival studies. Viability of 24 h post infection is significantly reduced by treatment of mice with Hap2 antibody, Hap2 and Hap5 antibody mix or gentamycin. N=6 per group. *P<0.01 and +P<0.05 lower for indicated group than for PBS negative control. Dashed line represents detection limit of viable count assay within blood.
Figure 5 shows the survival times of mice in the pulmonary infection model. N=6 per group. *P<0.01 and +P<0.05 longer for indicated group than for PBS negative control.
Figure 6 shows bacterial loads from tail bleeds taken at 24 h post-infection from survival studies and bacteriology studies combined. N=12 per group, *P<0.01 and +P<0.05 lower for indicated group than for PBS negative control. Dashed line represents detection limit of viable count assay within blood.
Figure 7 shows bacterial loads from lung airways (BAL fluid) at 12h post-infection N=6 per group. *P<0.01 and +P<0.05 lower for indicated group than for negative PBS control. Dashed line represents detection limit of viable count assay within lung airways.
Figure 8 shows Bacterial loads in lung tissue at 24 h post-infection. N=6 per group, *P<0.01 lower for indicated group than for PBS negative control. Dashed line represents detection limit of viable count assay within lung tissue.

### Example 1: Generation of anti-AHL antibodies.

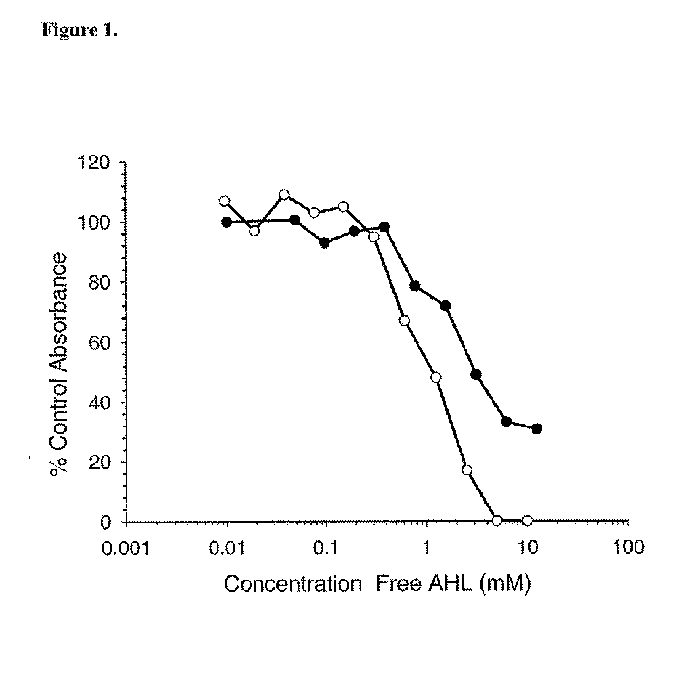

A naïve human antibody phage display library was screened against conjugates of the acyl-homoserine lactone dDHL (dodecanoyl homoserine lactone). Briefly, a derivative of dDHL including a carboxyl group at the end of the acyl chain was conjugated to the carrier proteins Bovine Serum Albumin (BSA) and Bovine Thyroglobulin (TG) using well known chemistry. The antibody library was screened (panned) against each conjugate alternately for three rounds, with those phage binding to conjugate being isolated, amplified, and used for the subsequent round. After the first round, all binding phage were recovered and amplified. During the second and third rounds, bound phage were eluted from the immobilised conjugate by incubation with a solution of free soluble native dDHL or BHL (Butyl-homoserine lactone). Monoclonal phage antibodies from round three were screened initially for binding to both AHL conjugates, and to carrier protein alone. Those clones binding only to the conjugated antigen were further screened for the ability to bind to free dDHL or free BHL by competitive binding ELISA. Two clones, designated Hap 2 and Hap 5, were isolated that could be inhibited in binding to dDHL-conjugate in the presence of free dDHL (Hap 2) and free BHL (Hap 5) (Figure 1).

### Example 2: Pseudomonas aeraginosa viability assay

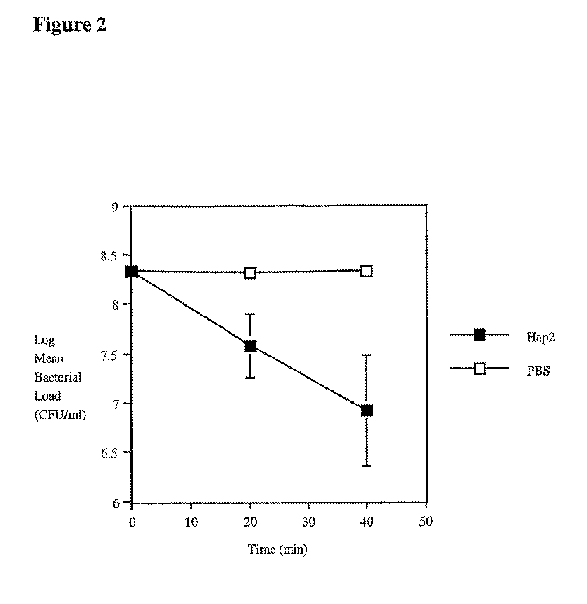

*Ps. aeruginosa* clinical isolate PA01 was passaged in female BALB/c mice (Charles River, approximately 30 days old) by intra-peritoneally injecting 50 microlitres of overnight PA01 broth culture containing approximately 5 x 10⁸ CFU as determined by serial plate dilutions. After 24 hours a blood sample was collected from the retro-orbital plexus and the PA01 isolate was recovered from the blood culture. Isolates were confirmed as being P. *aeruginosa* by Gram staining, colony morphology and pyocyanin production. Aliquots of bacteria were stored at -70°C and when required, were thawed rapidly, harvested by centrifugation and resuspended in 50 microlitres of sterile phosphate-buffered saline (PBS) or sterile PBS containing Hap2 antibody at a concentration of 37.5 µM. Aliquots of bacteria were placed on ice and serial dilutions in PBS were plated out on blood agar base plates at various time-points (0, 20, 40 minutes). Plates were incubated overnight at 37°C and colonies were counted to determine viable bacterial numbers at each time-point (Figure 2).

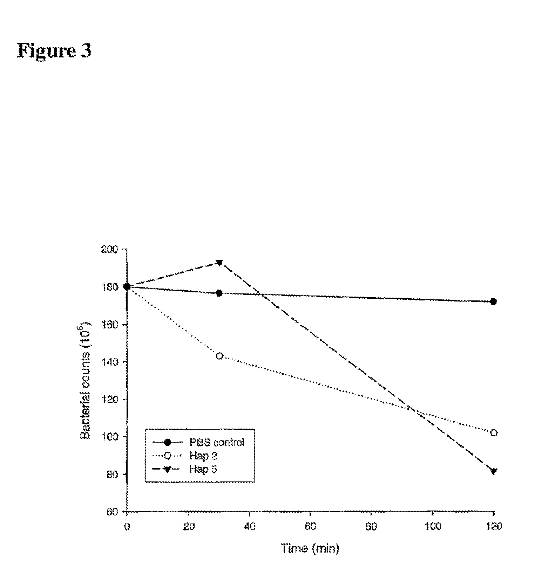

A second assay was developed using *P. aeruginosa* clinical isolate PA14 that had not been previously passaged in mice. A 5 ml culture of PA14 was grown overnight at 37°C in LB. Serial 10-fold dilutions to 10⁻⁶ were made in L broth, and 50 µL of dilution was added to 50 µL of PBS, antibody Hap2 (37.5 µM) or antibody Hap5 (15 µM). Samples were incubated on ice for various time periods and then immediately plated on brain heart infusion agar plates and incubated overnight at 37°C. Numbers of colonies were then counted for each plate (Figure 3).

### Example 3: Pseudomonas aeruginosa pulmonary infection model.

*Ps. aeruginosa* clinical isolate PA01 was passaged in female BALB/c mice (Charles River, approximately 4 weeks old) by intraperitoneally injecting 50 microlitres of overnight PA01 broth culture containing approximately 5 x 10⁸ CFU as determined by serial plate dilutions. After 24 hours a blood sample was collected from the retro-orbital plexus and the PA01 isolate was recovered from the blood culture. Isolates were confirmed as being P. *aeruginosa* by Gram staining, colony morphology and pyocyanin production. Aliquots of bacteria were stored at -70°C and when required, were thawed rapidly, harvested by centrifugation and resuspended in sterile phosphate-buffered saline (PBS). Serial dilutions of bacteria in PBS were plated out on blood agar base plates for viable cell enumeration. Plates were incubated overnight at 37°C and colonies were counted to determine viable bacterial numbers.

For the first pulmonary infection experiment, survival and bacteraemia were monitored over a period of one week. Six adult female BALB/c mice (approx. 4 weeks old) per treatment group were lightly anaesthetized using 2.5 % (v/v) halothane. An infectious dose of 1.0 x 10⁷ CFU PA01 resuspended in sterile PBS or PBS containing antibodies was administered into the nares in a total volume of 50 µl The infectious dose was confirmed by plating out serial dilutions on blood agar base plates for viable cell enumeration. Five treatment groups were monitored; the first group were infected with PA01 suspended in PBS alone; the second group were infected with PA01 mixed with 37.5 µM Hap 2 in PBS; the third were infected with PA01 mixed with 15 µM Hap 5 in PBS; the fourth were infected with PA01 mixed with the anti-AHL antibodies Hap 2 and Hap 5; and the final group was infected with PA01 mixed with PBS and given a course of subcutaneous injections of 0.2 mg gentamycin (first injection 2 h post-infection then with subsequent s/c boosts twice a day for 3 days). For the groups 2 and 3, 37.5 µM Hap 2 or 15 µM Hap 5 antibodies were mixed with the infectious dose immediately prior to intranasal infection (e.g. 10 µl bacteria plus 40 µl antibody). For group 4, 10 µl bacteria were mixed with 20 µl of 37.5 µM Hap 2 and 20 µl of 15 µM Hap 5.

At 2 h post infection mice were given an intravenous boost of PBS or PBS containing antibodies by directly injecting a total volume 50 µl into a tail vein.

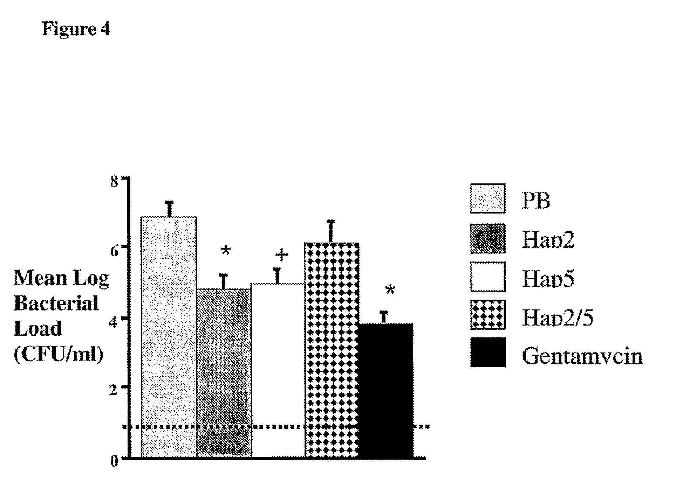

At 24 h, a small volume of blood was removed from the tail vein of each mouse using a 1 ml insulin syringe (12.7 mm) for determination of bacteraemia using viable cell enumeration on BAB plates (Figure 4). Treatment with either Hap2, Hap 2 mixed with Hap5 or gentamycin significantly reduced the levels of viable P. *aeruginosa* within the bloodstream at 24 h.

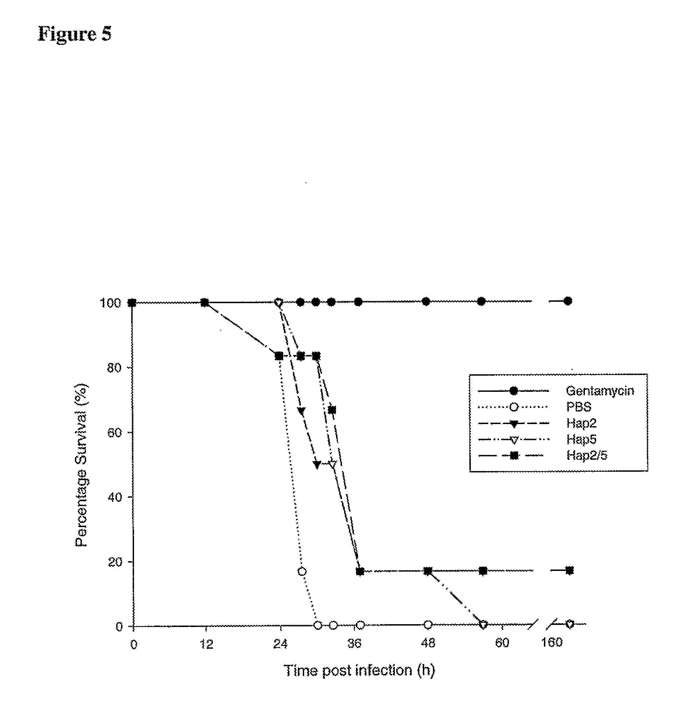

In order to determine experimental endpoints, symptoms (weights, condition, behaviour) were monitored frequently for 168 h post infection, and mice were culled prior to reaching, or upon reaching, a moribund state. The post-infection times at which each animal was actively culled were recorded and used as a measure of "survival". Animals that survived the 7 days of the infectious process were assigned an arbitrary survival time of 168 h for statistical analysis (Figure 5, Table 1). All infected mice lost weight during the first 3 days post-infection, but animals that survived the bacterial challenge then recovered and their weight returned to baseline by 1 week post infection. This trend was not affected by treatment with any of the antibodies or gentamycin. Survival times of the mice were significantly increased by treatment of the mice with Hap 5, Hap 2/Hap5 or gentamycin. One mouse from the Hap 2 and Hap2/5 treatment groups survived infection.

**Table 1**

| Treatment | Survival times (h) | | | | | |
|---|---|---|---|---|---|---|
| PBS | 24 | 27.5 | 27.5 | 27.5 | 27.5 | 30 |
| Hap 2 | 27.5 | 27.5 | 30 | 37 | 37 | S |
| Hap 5 | 27.5 | 32.5 | 32.5 | 37 | 37 | 57+ |
| Hap 2/5 | 24 | 32.5 | 37 | 37 | 37 | S+ |
| Gentamycin | S | S | S | S | S | S |

Table 1. Shows the survival times of mice in the pulmonary infection model. N=6 per group, *P<0.01 and +P<0.05 longer for indicated group than for PBS negative control, S= survived infection.

### Example 4: Effect of antibody on bacterial load

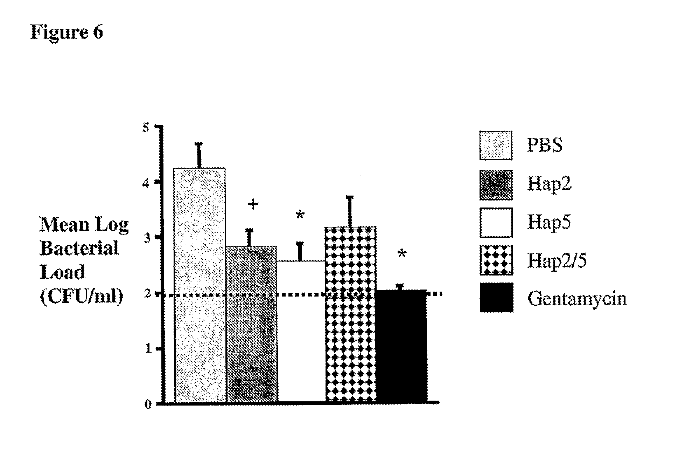

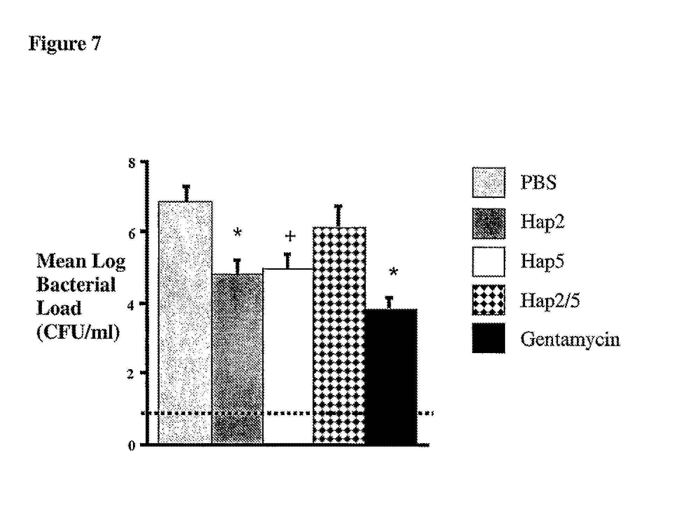

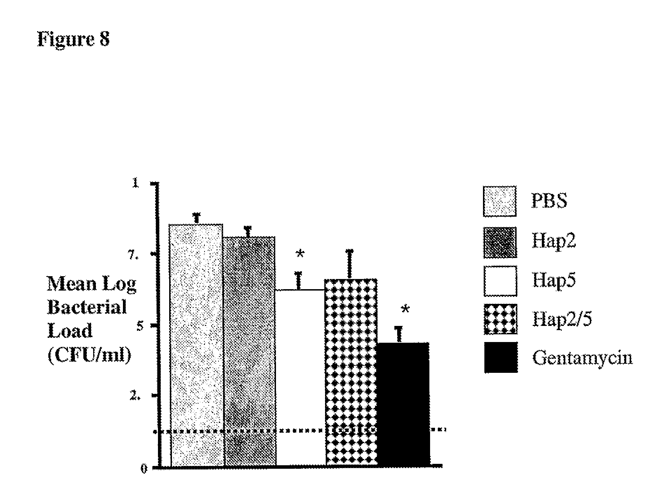

Five treatment groups of 6 mice were infected as described above, and given identical regimes of PBS, PBS plus antibodies or gentamycin. Two time-points (12 h and 24 h) were selected for analysis of bacteriology in blood (Figure 6), bronchoalveolar lavage (BAL) fluid (Figure 7), and lung tissue (Figure 8). Data from blood analysis were combined with that from the survival experiment (Example 2) in order to increase statistical validity. In this case viability of *P. aeruginosa* in the bloodstream was significantly reduced by treatment with Hap 2, Hap 5, or gentamycin.

Bacterial loads in BAL fluid and blood were determined by viable cell enumeration using serial dilutions on BAB plates as before. Viability of *P. aeruginosa* in the lung airways (BAL fluid) 12 h postinfection is significantly reduced by treatment of mice with Hap 2, Hap 5, or gentamycin.

Bacterial loads in lung tissue were determined by removing, weighing and homogenizing the lungs in 5 ml sterile PBS using an electric tissue homogenizer, then plating out serial dilutions on BAB plates for viable cell enumeration. Similar trends in the reduction of bacterial load were observed in the lung tissue examined 24 h postinfection (Figure 8), although only the Hap5 and gentamycin groups achieved statistically significant reductions in bacterial loads.

Geometric means of all results were calculated and are displayed along with ±1 standard error of the mean (SEM). Survival assays were analysed using Mann-Whitney U tests; all other data were analysed with Student's *t* test, and a P value of <0.05 was considered statistically significant.

### References cited

| **U.S. Patent Documents** | | |
|---|---|---|
| 6,309,651 | October 2001 | Frank et al. |

| **Other Patent Documents** | | |
|---|---|---|
| WO 01/26650 | April 2001 | University of Nottingham |
| WO 01/74801 | October 200 | University of Nottingham |
| WO 92/01047 | October 2001 | Bonnert et al. |

### Other References

William et al., 1996 Microbiol-UK 142: 881-888
Stintzi et al., 1998 FEMS Microbiol Lett. 166 (2): 341-345
Glessner et al., 1999 J. Bacteriol. 181 (5): 1623-1629
Brint and, Ohman 1995 J. Bacteriol. 177 (24): 7155-7163
Reimmann et al., 1997 Mol. Microbiol. 24 (2): 309-319
Winzer et al., 2000 J. Bacteriol. 182 (22): 6401-6411
Gambello and Iglewski 1991 J. Bacteriol. 173 (9): 3000-3009
Latifi et al., 1995 Mol. Microbiol 17 (2): 333-343
Passador et al., 1993 Science 260: 1127-1130
Pearson et al., 1994 Proc. Natl. Acad. Sci. USA. 91 (1): 197-201
Winson et al., 1995 Proc. Natl. Acad. Sci. USA. 92 (20): 9427-9431
Pesci et al., 1997 J. Bacteriol. 179 (10): 3127-3132
Toder et al., 1991 Mol. Microbiol. 5 (8): 2003-2010
Gambello et al., 1993 Infect. Immun. 61 (4): 1180-1184
Ochsner et al., 1994 J. Bacteriol. 176,2044-2054
Pearson et al., 1995 Proc. Natl. Acad. Sci. USA 92 (5) 1490-1494
Latifi et al., 1996 Mol. Microbiol 21 (6): 1137-1146
Winzer et al., 2000 J. Bacteriol. 182 (22): 6401-6411
Pesci et al, 1999 Proc, Natl. Acad. Sci. USA 96:11229-11234
Déziel et al., 2004 Proc. Natl. Acad. Sci. USA 101 (5): 1339-1344
Diggle et al., 2003 Molecular Microbiology 50 (1): 29-43
McGrath et al. 2004 FEMS Microbiol. Letters 230: 27-34
McKnight et al., 2000 J. Bacteriol. 182: 2702-2708
Burns et al., 2001 J. Infect. Dis. 183: 444-452
Singh et al., 2000 Nature 407: 762-764
Wu et al., 2000 Microbiology 146: 2481-2493
Wu et al., 2004 Microbes and Infection 6: 34-37
D'Argenio et al., 2002 Journal of Bacteriology 184 (23): 6481-6489
Guina et al., 2003 Proc. Natl. Acad. Sci. USA 100 (5): 2771-2776
Manefield et al., 1999 Microbiol. UK 145: 283-291
Tepletski et al., 2000 Mol. Plant Microbe. Interact., 13, 637-648
Kohler and Milstein, 1975 Nature 256: 495-497
Roitt et al, Immunology second edition (1989), Churchill Livingstone, London.
Dougall et al., 1994 TibTech 12: 372-379
McCafferty et al., 1990 Nature 348: 552-554
Huston et al., 1993 Int. Rev. Immunol., 10: 195-217

Aspects and features of the present disclosure are set out in the following numbered clauses which contain the subject matter of the claims of the parent application as filed.
1. A method of causing autolysis of a population of gram-negative bacteria, said method comprising administration to the population of an antibody to a lactone or lactone-derived signal molecule secreted by gram-negative bacteria so as to cause an imbalance in the ratio of homoserine lactone (HL) signal molecule to quinolone signal (QS) signal molecule in the environment of the population of the gram-negative bacteria.
2. A method of clause 1, in which the homoserine lactone (HL) signal molecule is a homoserine lactone molecule with a formula selected from the group consisting of: 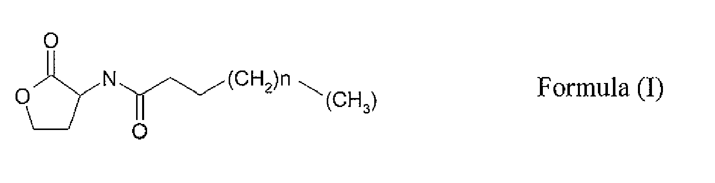 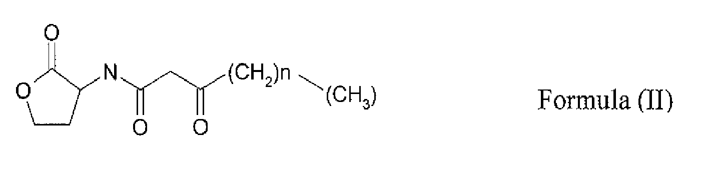 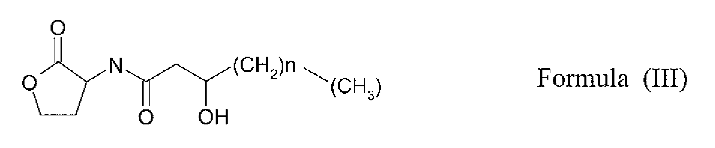 where n = 0 to 12.
3. A method of clause 2, in which the homoserine lactone molecule of general formula (1) is *N*-butanoyl-L-homoserine lactone (BHL) where n = 0, N-dodecanoyl-L-homoserine lactone (dDHL) where n = 8 and *n*-tetradecanoyl-L-homoserine lactone (tDHL) where n = 10.
4. A method of clause 2, in which the homoserine lactone molecule of general formula (II) is *N*-(-3-oxododecanoyl)-L-homoserine lactone (OdDHL) where n = 8 or *N*-(-3-oxohexanoyl)-L-homoserine lactone (OHHL) where n = 2.
5. A method of clause 2, in which the homoserine lactone molecule of general formula (III) is *N*-(-3-hydroxybutanoyl)-L-homoserine lactone (HBHL) where n = 0.
6. A method of clause 2, in which the lactone signal molecule is OdDHL and/or BHL.
7. A method of any preceding clause, in which the guinolone signal (QS) signal molecule is a molecule of general formula (IV): 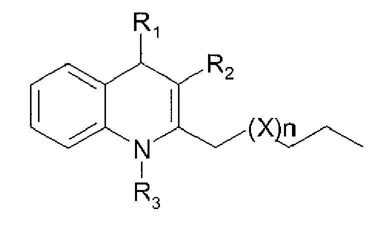
   where n is 1 to 7,
   R₁ is =O, or -H,
   R₂ is -OH, or -H, and
   R₃ is -H, or alternatively, the nitrogen atom (N) is unsubsituted.
8. A method of clause 7, in which the quinolone signal molecule of general formula (IV) is 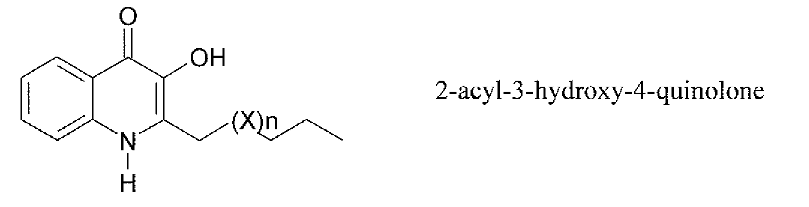
9. A method of clause 8, in which the 2-acyl-3-hydroxy-4-quinolone is 2-heptyl-3-hydroxy-4-quinolone 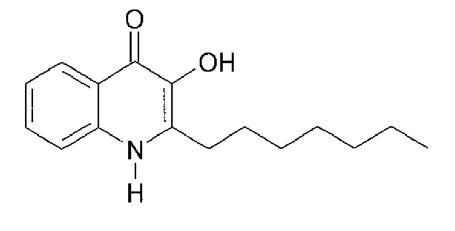
10. A method of any preceding clause, in which the gram negative bacteria is *Pseudomonas aeruginosa* and the ratio of bacterial signal molecules is acyl-homoserine lactone (AHL) signal molecule of formula (I) to *Pseudomonas* quinolone signal (PQS) signal molecule.
11. A method of any preceding clause, in which the antibodies are monoclonal or polyclonal antibodies, or fragments thereof.
12. A method of clause 11 in which the antibody fragments are single chain antibody fragments (scAbs).
13. A method of clause 12, in which the single-chain antibodies (scAbs) are G3H5, G3B12, G3G2 and/or G3H3 deposited as NCIMB-41167, NCIMB-41168, NCIMB-41169, NCIMB-41170, respectively.
14. A method for the treatment of an infection of gram-negative bacteria in a subject, said method comprising administration to the subject of an antibody to a lactone or lactone-derived signal molecule secreted by gram-negative bacteria so as to cause an imbalance in the ratio of homoserine lactone (HL) signal molecule to quinolone signal (QS) signal molecule in the environment of the gram-negative bacteria.
15. A method of clause 14, in which the homoserine lactone (HL) signal molecule is a homoserine lactone molecule with a formula selected from the group consisting of: 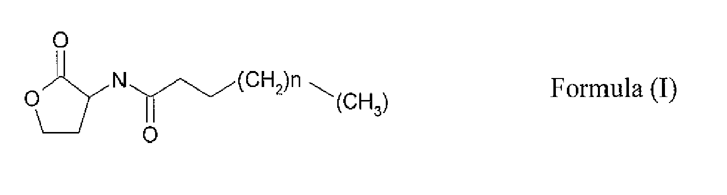 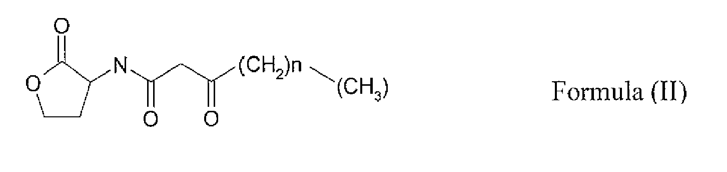 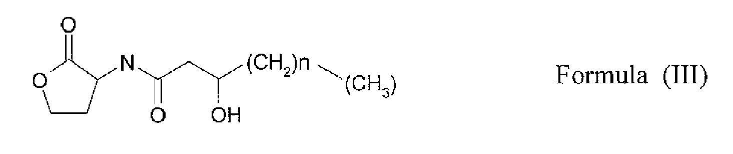 where n = 0 to 12.
16. A method of clause 15, in which the homoserine lactone molecule of general formula (I) is *N*-butanoyl-L-homoserine lactone (BHL) where n = 0, *N*-dodecanoyl-L-homoserine lactone (dDHL) where n = 8 and *n*-tetradecanoyl-L-homoserine lactone (tDHL) where n = 10.
17. A method of clause 15, in which the homoserine lactone molecule of general formula (II) is *N*-(-3-oxododecanoyl)-L-homoserine lactone (OdDHL) where n = 8 or *N*-(-3-oxohexanoyl)-L-homoserine lactone (OHHL) where n = 2.
18. A method of clause 15, in which the homoserine lactone molecule of general formula (III) is *N*-(-3-hydroxybutanoyl)-L-homoserine lactone (HBHL) where n = 0.
19. A method of clause 15, in which the lactone signal molecule is OdDHL and/or BHL.
20. A method of any one of clauses 14 to 19, in which the quinolone signal (QS) signal molecule is a molecule of general formula (IV): 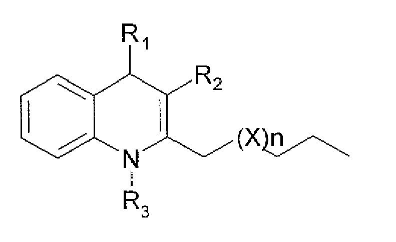
   where n is 1 to 7,
   R₁ is =O, or -H,
   R₂ is -OH, or -H, and
   R₃ is -H, or alternatively, the nitrogen atom (N) is unsubsituted.
21. A method of clause 20, in which the quinolone signal molecule of general formula (IV) is 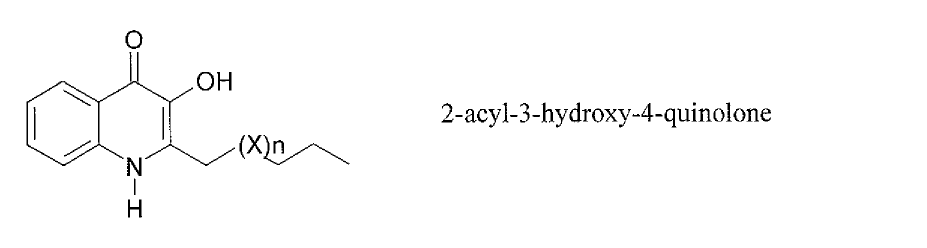
22. A method of clause 21, in which the 2-acyl-3-hydroxy-4-quinolone is 2-heptyl-3-hydroxy-4-quinolone 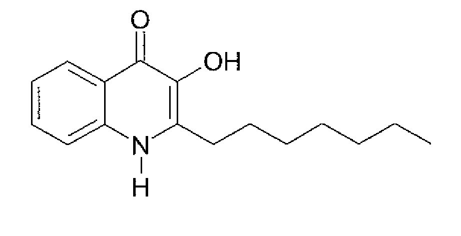
23. A method of any one of clauses 14 to 22, in which the gram negative bacteria is *Pseudomonas aeruginosa* and the ratio of bacterial signal molecules is acyl-homoserine lactone (AHL) signal molecule of formula (I) to *Pseudomonas* quinolone signal (PQS) signal molecule.
24. A method of any one of clauses 14 to 23, in which the antibodies are monoclonal or polyclonal antibodies, or fragments thereof.
25. A method of clause 24 in which the antibody fragments are single chain antibody fragments (scAbs).
26. A method of clause 25, in which the single-chain antibodies (scAbs) are G3H5, G3B12, G3G2 and/or G3H3 deposited as NCIMB-41167, NCIMB-41168, NCIMB-41169, NCIMB-41170, respectively.
27. An antibody to a lactone or lactone-derived signal molecule secreted by gram-negative bacteria for use in causing autolysis of gram-negative bacteria.
28. The antibody for use of clause 27, in which the lactone or lactone-derived signal molecule is a homoserine lactone molecule with a formula selected from the group consisting of: 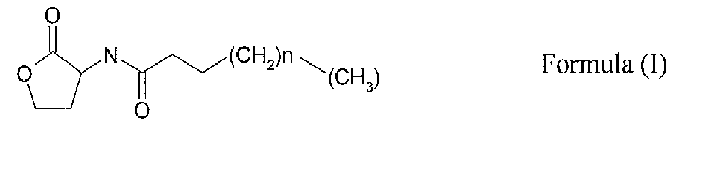 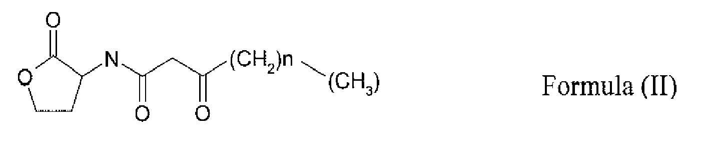 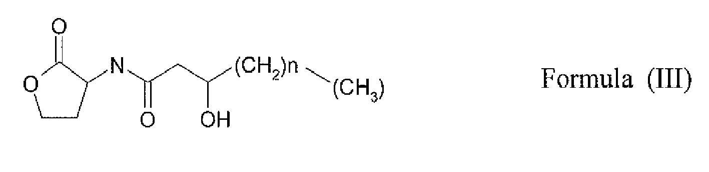 where n = 0 to 12.
29. The antibody for use of clause 28, in which the homoserine lactone molecule of general formula (1) is *N*-butanoyl-L-homoserine lactone (BHL) where n = 0, *N-*dodecanoyl-L-homoserine lactone (dDHL) where n = 8 and *n*-tetradecanoyl-L-homoserine lactone (tDHL) where n = 10.
30. The antibody for use of clause 28, in which the homoserine lactone molecule of general formula (II) is *N*-(-3-oxododecanoyl)-L-homoserine lactone (OdDHL) where n = 8 or *N*-(-3-oxohexanoyl)-L-homoserine lactone (OHHL) where n = 2.
31. The antibody for use of clause 28, in which the homoserine lactone molecule of general formula (III) is *N*-(-3-hydroxybutanoyl)-L-homoserine lactone (HBHL) where n = 0.
32. The antibody for use of clause 28, in which the lactone signal molecule is OdDHL and/or BHL.
33. The antibody for use of any one of clauses 27 to 32, in which the quinolone signal (QS) signal molecule is a molecule of general formula (IV): 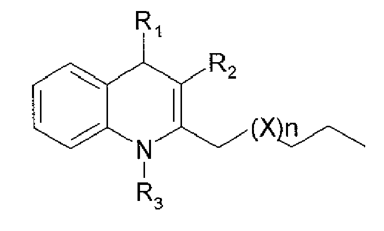
   where n is 1 to 7,
   R₁ is =O, or -H,
   R₂ is -OH, or -H, and
   R₃ is -H, or alternatively, the nitrogen atom (N) is unsubsituted.
34. The antibody for use of clause 33, in which the quinolone signal molecule of general formula (IV) is 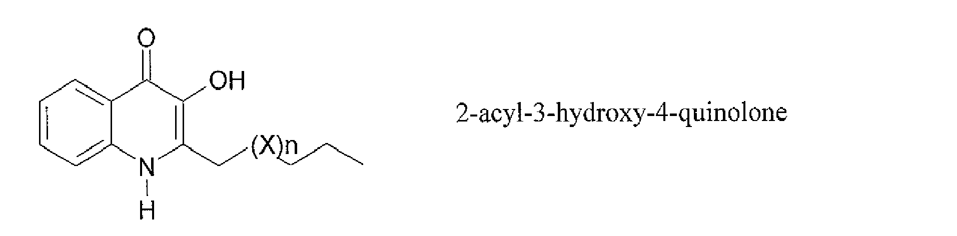
35. The antibody for use of clause 34, in which the 2-acyl-3-hydroxy-4-quinolone is 2-heptyl-3-hydroxy-4-quinolone 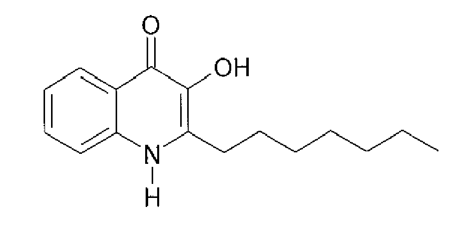
36. The antibody for use of any one of clauses 27 to 35, in which the gram negative bacteria is *Pseudomonas aeruginosa* and the ratio of bacterial signal molecules is acyl-homoserine lactone (AHL) signal molecule of formula (I) to *Pseudomonas* quinolone signal (PQS) signal molecule.
37. The antibody for use of any one of clauses 27 to 36, in which the antibodies are monoclonal or polyclonal antibodies, or fragments thereof.
38. The antibody for use of clause 37 in which the antibody fragments are single chain antibody fragments (scAbs).
39. The antibody for use of clause 38, in which the single-chain antibodies (scAbs) are G3H5, G3B12, G3G2 and/or G3H3 deposited as NCIMB-41167, NCIMB-41168, NCIMB-41169, NCIMB-41170, respectively.
40. The use of an antibody to a lactone or lactone-derived signal molecule secreted by gram-negative bacteria in the preparation of a medicament for the treatment of an infection of gram-negative in a subject, in which the antibody causes autolysis of the gram-negative bacteria which infect said subject.

## Claims

1. A method of causing autolysis of a population of gram-negative bacteria, said method comprising administration to the population of an antibody to a lactone or lactone-derived signal molecule secreted by gram-negative bacteria so as to cause an imbalance in the ratio of homoserine lactone (HL) signal molecule to quinolone signal (QS) signal molecule in the environment of the population of the gram-negative bacteria.

2. An antibody to a lactone or lactone-derived signal molecule secreted by gram-negative bacteria for use in causing autolysis of gram-negative bacteria.

3. A method as claimed in claim 1, or an antibody to a lactone or lactone-derived signal molecule secreted by gram-negative bacteria for use as claimed in claim 2, in which the homoserine lactone (HL) signal molecule is a homoserine lactone molecule with a formula selected from the group consisting of: 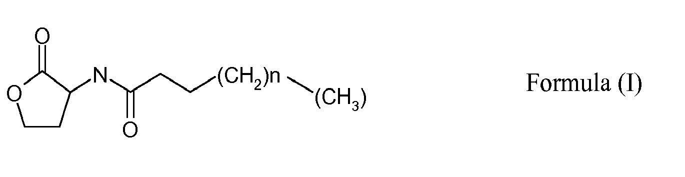 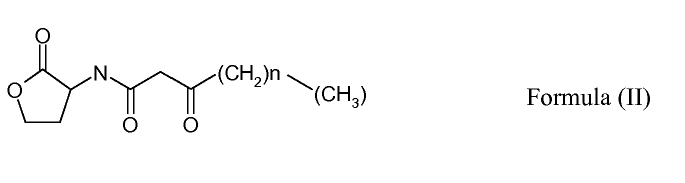 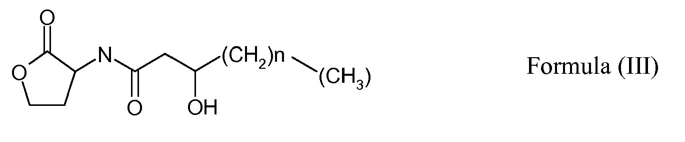 where n = 0 to 12.

4. A method as claimed in claim 3, or an antibody to a lactone or lactone-derived signal molecule secreted by gram-negative bacteria for use as claimed in claim 3, in which the homoserine lactone molecule of general formula (I) is N-butanoyl-L-homoserine lactone (BHL) where n = 0, *N*-dodecanoyl-L-homoserine lactone (dDHL) where n = 8 and n-tetradecanoyl-L-homoserine lactone (tDHL) where n = 10.

5. A method as claimed in claim 3, or an antibody to a lactone or lactone-derived signal molecule secreted by gram-negative bacteria for use as claimed in claim 3, in which the homoserine lactone molecule of general formula (II) is *N*-(-3-oxododecanoyl)-L-homoserine lactone (OdDHL) where n = 8 or N-(-3-oxohexanoyl)-L-homoserine lactone (OHHL) where n = 2.

6. A method as claimed in claim 3, or an antibody to a lactone or lactone-derived signal molecule secreted by gram-negative bacteria for use as claimed in claim 3, in which the homoserine lactone molecule of general formula (III) is *N*-(-3-hydroxybutanoyl)-L-homoserine lactone (HBHL) where n = 0.

7. A method as claimed in claim 3, or an antibody to a lactone or lactone-derived signal molecule secreted by gram-negative bacteria for use as claimed in claim 3, in which the lactone signal molecule is OdDHL and/or BHL.

8. A method as claimed in claim 1 or any one of claims 3 to 7, or an antibody to a lactone or lactone-derived signal molecule secreted by gram-negative bacteria for use as claimed in any one of claims 2 to 7, in which the quinolone signal (QS) signal molecule is a molecule of general formula (IV): 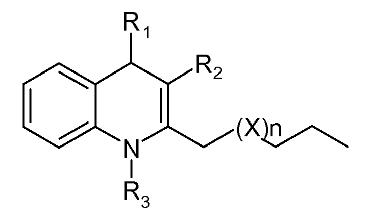
where n is 1 to 7,
R₁ is =O, or -H,
R₂ is -OH, or -H, and
R₃ is -H, or alternatively, the nitrogen atom (N) is unsubsituted.

9. A method as claimed in claim 8, or an antibody to a lactone or lactone-derived signal molecule secreted by gram-negative bacteria for use as claimed in claim 8, in which the quinolone signal molecule of general formula (IV) is

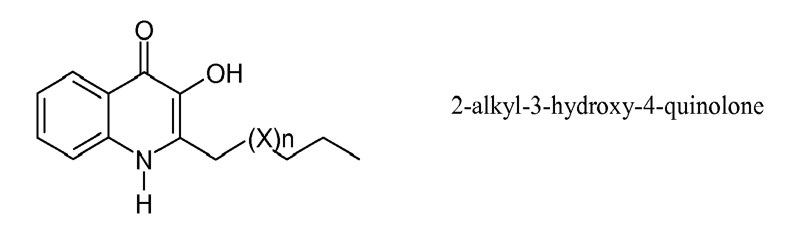

10. A method as claimed in claim 9, or an antibody to a lactone or lactone-derived signal molecule secreted by gram-negative bacteria for use as claimed in claim 9, in which the 2-alkyl-3-hydroxy-4-quinolone is 2-heptyl-3-hydroxy-4-quinolone

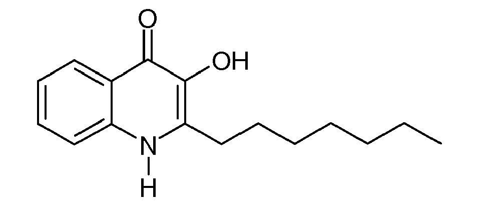

11. A method as claimed in claim 1 or any one of claims 3 to 10, or an antibody to a lactone or lactone-derived signal molecule secreted by gram-negative bacteria for use as claimed in any one of claims 2 to 10, in which the gram negative bacteria is *Pseudomonas aeruginosa* and the ratio of bacterial signal molecules is acyl-homoserine lactone (AHL) signal molecule of formula (I) to *Pseudomonas* quinolone signal (PQS) signal molecule.

12. A method as claimed in claim 1 or any one of claims 3 to 10, or an antibody to a lactone or lactone-derived signal molecule secreted by gram-negative bacteria for use as claimed in any one of claims 2 to 10, in which the antibodies are monoclonal antibodies, or fragments thereof.

13. A method as claimed in claim 12, or an antibody to a lactone or lactone-derived signal molecule secreted by gram-negative bacteria for use as claimed in claim 12, in which the antibody fragments are single chain antibody fragments (scAbs).

14. A method as claimed in claim 13, or an antibody to a lactone or lactone-derived signal molecule secreted by gram-negative bacteria for use as claimed in claim 13, wherein the single-chain antibodies (scAbs) are G3H5, G3B12, G3G2 and/or G3H3 deposited as NCIMB-41167, NCIMB-41168, NCIMB-41169, NCIMB-41170, respectively.

15. An antibody to a lactone or lactone-derived signal molecule secreted by gram-negative bacteria for use in the treatment of an infection of gram-negative in a subject, in which the antibody causes autolysis of the gram-negative bacteria which infect said subj ect.
